Europäisches Patentamt

European Patent Office

Office européen des brevets

(19)

(11) Publication number: **0 228 710 B1**

# EUROPEAN PATENT SPECIFICATION

(12)

(45) Date of publication of patent specification: **10.04.91**   (51) Int. Cl.⁵: **D21H  27/00, C08J  7/00**

(21) Application number: **86118135.2**

(22) Date of filing: **30.12.86**

(54) Method for producing active agent-containing laminated paper.

(30) Priority: **30.12.85 JP 298276/85**

(43) Date of publication of application:
**15.07.87 Bulletin  87/29**

(45) Publication of the grant of the patent:
**10.04.91 Bulletin  91/15**

(84) Designated Contracting States:
**BE DE FR GB IT NL**

(56) References cited:
**DE-A- 3 503 666**
**GB-A- 986 558**
**US-A- 1 368 547**
**US-A- 3 853 592**
**US-A- 4 533 435**

**ABSTRACT BULLETIN OF THE INSTITUTE OF
PAPER CHEMISTRY, vol. 55, no. 9, March
1985, page 1071, abstract no. 10104, Appleton, Wisconsin, US**

**CHEMICAL ABSTRACTS, vol. 97, no. 26, 27th
December 1982, page 126, abstract no.
218361t, Columbus, Ohio, US**

(73) Proprietor: **Nippon Petrochemicals Co., Ltd.
Miyuki Building, 3-1 Uchisaiwai-cho 1-chome
Chiyoda-ku
Tokyo(JP)**

(72) Inventor: **Saitoh, Hachiro
19, Yasuuracho 3-chome
Yokosuka-shi Kanagawa-ken(JP)**

(74) Representative: **Strehl, Schübel-Hopf, Groening
Maximilianstrasse 54 Postfach 22 14 55
W-8000 München 22(DE)**

## Description

BACKGROUND OF THE INVENTION

### (1) Field of the Invention

This invention relates to a method for producing active agent-containing laminated paper. More particularly, the invention relates to a method for producing active agent-containing laminated paper in which method undesirable releasing and thermal deterioration of active agents can be well avoided and the adhesive strength of obtained laminated paper is quite high.

### (2) Description of the Prior Art

In the conventional art, perfumes, antifungal agents, rust inhibitors, insecticides, repellents and the like (hereinafter referred to as "active agents"), especially volatile ones, are generally used in the forms of powder, tablets or solutions in water or alcohol. In the case of a solution, paper or cloth is coated or impregnated with the solution to obtain fragrant paper, rust inhibitive paper and so forth.

There have been, however, several problems in that the effect of an active agent is liable to be lost in a short period of time and the heat-sealable property and water-proofness of active agent-containing paper are not satisfactory.

For this reason, several polyolefin compositions are proposed which are prepared by mixing active agents into plastics such as polyolefin by using carriers so as to regulate the rate of releasing of the active agents to be slow and constant.

For example, in GB-A- 1,538,085, it is proposed that a master batch of perfume-containing ethylene-vinylacetate copolymer is blended into polyethylene to obtain a composition and this composition is used for refuse bags and so forth.

Similar examples are disclosed in many patent specifications such as U.S.-A- 4,095,031 (corres. to JP-A- 53-98352); CA-A- 844,014; U.S.-A- 3,553,296; and JP-A- 56-121560. These compositions are used singly or in combination with other plastics to form film materials and injection molded goods.

Furthermore, in the present status of industries, there are few instances of laminated materials that are made of the combination of a composition of active agent and polyolefin with a substrate material made of other plastics films or paper. Only in the field of volatile rust inhibitors, a few patents are known (U.S.-A-282,980 and US-A- 3,080,211 and JP-A- 52-13988).

The laminated materials described in these patents are produced by the steps such that a volatile rust inhibitor is dissolved in an emulsified resin or a solvent and the obtained solution is applied to a film to obtain a covering film. The covering film is then laminated with a base film. Otherwise, a plastics layer containing a rust inhibitor is bonded firmly to a base film.

JP-A-84 43 082 discloses a method for gluing a surface protected paper to a substrate, in which water is sprayed onto the back of the paper having a coating layer of a pressure sensitive adhesive (which adhesive may be a polymer, such as EVA). The water treated paper is then pressed against a plastic or metal sheet as a surface protective layer.

JP-A-82 29 600 for example relates to the production of anti-corrosive paper having oil repellency. This paper is produced by laminating paper with a polyethylene film and impregnating the paper side with dicyclohexylamine nitrite. In this anti-corrosive paper, the carrier for the active agent is the paper, since the polyethylene film is a barrier layer which is incompatible with the polar compound dicyclohexylamine nitrite.

While, perfumes are used not only for cosmetics, soaps, detergents, tooth pastes, medicines, bath agents, cakes, chewing gum, alcoholic liquors and refreshing beverages but also for daily life with the recent change of life style into a variety of the mode of life enjoying comfortable fragrances.

In order to meet the above requirement, the inventors have proposed posters, postcards, bookmarkers, labels, greeting cards, interior wall materials and packaging materials that are made of laminated materials of a perfume-containing polyolefin composition and a substrate material such as paper.

These laminated materials are generally obtained by firstly preparing an active agent-containing film and laminating it with a substrate material such as paper.

In the common lamination process for producing laminated materials, several kinds of substrate materials of papers such as quality paper or kraft paper, plastics film, regenerated cellulose film and metal foil are coated by molten polyethylene by extrusion lamination, thereby producing various kinds of wrapping or packaging materials and so forth utilizing the advantageous properties of the combination of a substrate material and a plastics material such as water-proofness, moisture-proofness, chemical resistance, softness, heat-sealable property, etc.

The adhesive bonding mechanisms in the above lamination process are classified into two kinds, i.e., mechanical adhesive bonding and chemical adhesive bonding. The former mechanical adhesive bonding is applied to porous substrate materials such as cloth and paper, in which the molten polymer extruded at high a temperature infiltrates into fine pores among fibers and it is then solidified by cooling providing adhesive bonding between a plastics material and a substrate material.

The latter chemical adhesive bonding utilizes the chemical intermolecular force and it is applied to the materials having smooth surfaces such as regenerated cellulose film, aluminum foil and plastics film or those having no functional groups. In order to obtain satisfactory bonding force in the chemical adhesive bonding, it is necessary that the substrate material is subjected to primer treatment, and it is often treated by corona discharge.

Meanwhile, it is also desirable that the polyethylene used for extrusion coating is activated. As the method for this activation, it is a general practice that polyethylene is extruded at higher temperatures so as to increase oxygen-containing polar groups (carbonyl groups) in molecules before it is brought into contact with a substrate material so as to provide sufficient adhesive strength.

In the former mechanical adhesive bonding, when the temperature of molten polymer is high, the viscosity of polymer is lowered to improve the infiltration into a substrate material and the adhesive strength is also improved. Therefore, the lamination is done at possibly high temperatures as far as the polymer is not decomposed.

Accordingly, the generally used low density polyethylene is melted and kneaded by being heated to, at the lowest, 280 to 310°C in the conventional lamination process. In this process, it is necessary that the polyethylene is not decomposed and does not give off any offensive odor. In the industrial practice, however, partial decomposition is caused to occur with giving off irritating smell of decomposed product and much smoke is emitted during the processing.

The conventional extrusion lamination process like this is accompanied by serious problems such as the releasing, thermal decomposition and thermal deterioration of active agent by high temperatures. In addition, due to the use of a volatile active agent, sufficient adhesive strength between laminated layers cannot be obtained.

The present invention has been accomplished in order to solve the problems inherent in the above-described conventional art.

It is, therefore, the primary object of the present invention to provide a method for producing active agent-containing laminated paper which is free from the disadvantages in the conventional art.

Another object of the present invention is to provide a method for producing active agent-containing laminated paper in which the releasing and thermal deterioration of active agents are well avoided.

Still a further object of the present invention is to provide an improved method for producing active agent-containing laminated paper, by which method laminated paper with a high adhesive strength between layers of laminated materials, can be obtained.

These objects can be achieved as claimed in claim 1. The dependent claims refer to preferred embodiments of the invention.

In accordance with the present invention, the method for producing active agent-containing laminated paper comprises impregnating paper with an active agent and is characterized by the steps of:

applying and impregnating a liquid active agent to the paper layer of a laminated paper composed of the paper layer and a thermoplastic polymer layer which is compatible with said active agent and is laminated to one side of said paper layer; and

winding or overlaying together said impregnated laminated paper for a predetermined period of time;

thereby causing at least a part of said liquid active agent to shift and permeate into said thermoplastic polymer layer.

DETAILED DESCRIPTION OF THE INVENTION

In the present invention, as the thermoplastic polymers which are compatible with the active agents, several polymers such as petroleum resin, non-vulcanized rubber, polybutene, polyvinyl chloride resin, carboxyl group-containing ethylene copolymer, especially ethylene-vinyl acetate copolymer (EVA) and ethylene-ethylacrylate copolymer (EEA), are advantageously used. Among them, the ethylene-vinyl acetate copolymer is preferable. Furthermore, a mixture of them with an appropriate polyolefin can also be used.

The foregoing paper which is capable of being impregnated with an active agent is exemplified by common cellulosic paper, synthetic pulp paper, woven fabric and nonwoven fabric. These materials should not be soluble in an active agent or its solvent. The thickness of these materials may be suitably determined according to the purpose of use of the active agent-containing laminated paper, the kind and thickness of the foregoing thermoplastic polymer layer, and the aimed final concentration of an active agent in a product to be obtained. The average basis weight of the substrate paper is generally in the range of 20 to 500 g/m$^2$.

The active agents which are used for the impregnation of agent-acceptable paper are exemplified by perfumes, rust inhibitors, antifungal agents, antiseptic agents, germicides, mothproofing agents, rodenticides, deodorants, insecticides, repellents, and antifouling agents.

Perfumes are divided into two groups of natural perfumes and artificial perfumes. The natural perfumes are exemplified by animal perfumes of musk, civet, castreum and amber gris and vegetable perfumes consisting of essential oils are lavender oil, peppermint oil, lemon oil, orange oil, rose oil, camphor oil, sandalwood oil, and hinoki oil.

The artificial perfumes are exemplified by synthetic perfumes such as terpene compounds and aromatic compounds, isolated perfumes derived by fractional distillation from essential oils, and other various isolated perfumes, and formulated perfumes prepared by mixing synthetic perfumes and natural perfumes in compliance with practical uses. At least one of them is used in the method of the present invention.

Other active agents are further exemplified by deodorants such as "Fresh Shiraimatsu" (trademark, made by Shiraimatsu Shin'yaku Co., Ltd.), "Biodash" (trademark, made by Osaka Soda Co., Ltd.; Tokyo Seikagaku Kenkyusho Co., Ltd.), "Anico" (trademark, made by Minato Co., Ltd.); antifungal agents such as thiabendazole, vinazine and α-bromo cinnam aldehyde; germicides such as alcohols, formalin, salicylic acid, creosote, phenol, nitrofurazone, and nitrofurylacrylic acid amide; pyrethroid insecticides such as natural pyrethrin and synthetic pyrethrin-like compounds such as "Pinamin", "Permethrin", "Vaperthrin" (trademarks, all made by Sumitomo Chemical Co., Ltd.); insecticides such as DDT, BHC, drine-agents, parathion, DDVP, and PGP; rodenticides such as "Naramycin" (trademark, made by Tanabe Seiyaku Co., Ltd.), "Ramtarine" (trademark, made by Matsushita Electric Works Co., Ltd., "Kotomycin" (trademark, made by Osaka Kasei Co., Ltd.); repellents for dogs and cats such as lemongrass oil and tar of tobacco; volatile rust inhibitors of organic amine salts such as cyclohexylammonium nitrite and heterocyclic amines such as benzotriazole and methylbenzotriazole; antiseptic agents for foodstuffs such as sorbic acid and dehydroacetic acid; and antifouling agents such as phenylmercuric acetate, phenylmercuric oleate, copper naphthenate and copper oleate. These active agents can be used singly or in combination of two or more kinds in accordance with the purpose and utility.

The ratio of impregnation of the above active agents is preferably at least 0.005 part by weight relative to 100 parts by weight of a thermoplastic resin. If the impregnating ratio is less than 0.005 part by weight, the obtained product is not practically usable because the effect of active agent is too small.

These active agents can be used either singly or in combination of two or more kinds. In the case that an active agent is of solid, it is used in the form of a solution dissolved in a hardly-volatile solvent which has a good solubility to the active agent and a good compatibility with the thermoplastic polymer.

In the preparation of the active agent-containing laminated paper of the present invention, laminated paper consisting of paper which is capable of being impregnated with an active agent and at least a layer of a thermoplastic polymer which is compatible with said active agent, is prepared in the first place. In compliance with the form and objects of the final products, it is possible to overlay a layer of another material on the side opposite to the paper layer of the laminate material. For instance, when an agent-impermeable film is applied to the opposite side, the releasing of active agent to no purpose can be avoided during the preparation and use of the active agent-containing laminated paper of the invention.

The paper layer of the laminate is then applied and impregnated with a suitable quantity of the foregoing active agent and, after that, the impregnated laminated paper is wound when it is a web material, or overlaid together when it is a plurality of sheet materials, and preserved for a predetermined period of time. By the above procedure, the active agent impregnated into the paper layer is sufficiently shifted and permeated into the thermoplastic polymer layer. In this operation, the time required of the active agent to shift and permeate into the thermoplastic polymer depends upon the kinds of active agent and thermoplastic polymer, ambient temperature, the thicknesses of layers of the laminate, the pressure of winding of the laminate and other various factors. However, the optimum time length for permeation can be easily determined in accordance with the results of preliminary tests or sampling at appropriate time intervals. In the case that the application quantity of an active agent is too small, the agent can be applied directly to the side of thermoplastic polymer layer itself. On the other hand, when the quantity of the active agent is to large, undesirable fouling of apparatus such as guide rollers are sometimes caused to occur because the laminate with insufficient permeation passes through the process.

In the case that the active agent is a liquid agent such as perfume, it can be impregnated into a paper layer by applying it to to the surface of the paper layer. When the active agent is, however, a solid material such as a rust inhibitor, an insecticide or an untifungal agent, it can be impregnated by being dissolved into a proper volatile solvent or a odorless non-volatile sclvent. The term "liquid active agent" herein used covers these agents that are dissolved into solvents.

The active agent-containing laminated paper according to the present invention is then cut into a suitable shape in compliance with the aimed product. The cut laminated paper is temporarily applied with impermeable film or packaged to provide final products.

The above-mentioned impermeable film is exemplified by those made of thermoplastic resins such as acrylonitrile-rich copolymer, saponified ethylene-vinyl acetate copolymer, polyvinylidene chloride resin, polyester resins such as polyethylene terephthalate, polyamide resin and polycarbonate resin; paper coated with these polymeric resins; metal foils such as aluminum foil and tin foil; and regenerated cellulose film.

In the method of the present invention, it is not carried out that the film of thermoplastic polymer containing an active agent is previously formed and it is then laminated. However, laminated paper composed of at least one layer of paper which is capable of being impregnated with an active agent and a thermoplastic polymer layer which is compatible with said active agent is previously formed and the paper layer is then impregnated with a liquid active agent, thereby shifting and permeating the liquid active agent into the thermoplastic polymer layer. Accordingly, the active agent-containing laminated paper can be prevented from the influences of heat caused during the film formation of thermoplastic polymer and the lamination with paper, which fact avoids the releasing and deterioration of active agent by heating. In addition, the adhesive strength between the component layers of the laminated paper of the invention is quite high.

The present invention will be described in more detail with reference to several examples. In examples, active agent-containing laminated papers were prepared by the method of the present invention and by a conventional method, and the state of releasing of active agent and change of active agent were compared.

Example 1

Ethylene-vinyl acetate copolymer (EVA, made by Toyo Soda Manufacturing Co., Ltd., trademark: Ultrathene UE 632) was laminated to quality paper of about 50 g/m² in basis weight by extrusion lamination to obtain laminated paper having an about 30 μm thick EVA layer.

To the paper side of this laminated paper was applied about 2 g/m² of white rose perfume. It was rolled up intact and was stood still in a room for about 2 days. After that, it was unrolled and found the fact that the paper layer as well as the EVA layer were not wetted. Even though the contained liquid of about 2.5% by weight was not observed on the surfaces, the laminated paper was giving out fragrance from both side surfaces, which fragrance was maintained for about 6 months. The adhesive strength between the paper layer and the EVA layer was about 540 g/15 mm.

When the perfume was applied to the laminated paper, the released fragrance was not felt so strong and the tone of odor of the laminated paper was not different from that of the original perfume.

Comparative Example 1

EVA used in Example 1 was impregnated with about 8.9% by weight of white rose perfume and it was then formed into a perfume-containing film of about 30 μm thick by the conventional inflation method. During the film formation, the factory room was filled with strong smell of the perfume. After the perfumed film was left in a room for 2 days, the fragrance of the film was weaker than that of the laminated paper prepared in Example 1.

With extrusion lamination of 20 μm thick low density polyethylene (LDPE), the above perfume-containing film was sandwiched between the LDPE film and quality paper of about 50 g/m² in basis weight to obtain laminated paper.

Even though the laminated paper contained about 2.5% by weight of perfume, the fragrance of the perfume was weaker than that of the perfume-containing laminated paper prepared in Example 1 and the adhesive strength was as low as about 85 g/15 mm.

Comparative Example 2

EVA used in Example 1 and Comparative Example 1 was impregnated with about 7% by weight of white rose perfume. By the conventional extrusion lamination (temperature of extruded EVA: ca. 250°C, thickness: ca. 30 μm), it was then laminated with quality paper of about 50 g/m² in basis weight to obtain a perfume-containing laminated paper. During the film formation, the factory room was filled with strong smell of the perfume. The tone of the obtained perfume-containing laminated paper was quite different from the fragrance of the original perfume due to the thermal deterioration of the perfume. As compared with Example 1, the adhesive strength was also as lower as about 56 g/15 mm.

In the method for producing the active agent-containing laminated paper according to the present invention, laminated paper composed of a paper layer which is capable of being impregnated with an active agent and a thermoplastic polymer layer which is compatible with the active agent is firstly prepared and after that, the paper layer of the laminated paper is impregnated with a liquid active agent, thereby shifting and permeating the active agent into the thermoplastic polymer layer. Therefore, the active agent can be used effectively without suffering the undesirable releasing and deterioration by the heat of processing. Furthermore, it causes no pollution in a production site. Still further, the adhesive strength between laminated layers is good. Accordingly, the present invention is advantageous especially when a product that is impregnated with a volatile perfume or easily deteriorated perfume, is obtained.

## Claims

1. A method for producing active agent-containing laminated paper comprising impregnating paper with an active agent, characterized by the steps of:

   applying and impregnating a liquid active agent which can be dissolved in a solvent to the paper layer of a laminated paper composed of the paper layer and a thermoplastic polymer layer which is compatible with said active agent and is laminated to one side of said paper layer; and

   winding or overlaying together said impregnated laminated paper for a predetermined period of time;

   thereby causing at least a part of said liquid active agent to shift and permeate into said thermoplastic polymer layer.

2. The method for producing active agent-containing laminated paper of Claim 1, wherein said thermoplastic polymer which is compatible with an active agent, is a carboxyl group-containing ethylene copolymer or a composite of a carboxyl group-containing ethylene copolymer and other polymers.

3. The method for producing active agent-containing laminated paper of Claim 2, wherein said carboxyl group-containing ethylene copolymer is at least one member selected from the group consisting of ethylene-vinyl acetate copolymer and ethylene-ethylacrylate copolymer.

4. The method for producing active agent-containing laminated paper of any of the Claims 1 to 3, wherein said active agent is a volatile active agent.

5. The method for producing active agent-containing laminated paper of Claim 4, wherein said volatile active agent is at least one member selected from the group consisting of perfumes, anti-stink agents, deodorants, antifungal agents, antiseptic agents, germicides, rust inhibitors, mothproofing agents, insecticides, repellents, rodenticides, and antifouling agents.

## Revendications

1. Procédé de fabrication d'un papier stratifié contenant un agent actif comprenant l'imprégnation de papier par un agent actif, caractérisé par les phases de:

   application et imprégnation d'un agent actif liquide soluble dans un solvant à la couche papier d'un papier stratifié composé de la couche papier et d'une couche polymère thermoplastique compatible avec ledit agent actif et stratifiée sur une face de ladite couche papier et,

   enroulement ou superposition dudit papier stratifié imprégné pendant un laps de temps prédéfini,

   de manière à provoquer la migration d'au moins une partie dudit agent actif liquide et sa pénétration dans la couche polymère thermoplastique.

2. Procédé de fabrication de papier stratifié contenant un agent actif selon la revendication 1, dans lequel ledit polymère thermoplastique qui est compatible avec un agent actif est un copolymère d'éthylène contenant un groupe carboxyle ou un composite d'un copolymère d'éthylène contenant un groupe carboxyle et d'autres polymères.

3. Procédé de fabrication de papier stratifié contenant un agent actif selon la revendication 2, dans lequel le copolymère d'éthylène contenant un groupe carboxyle est au moins l'un des éléments choisis dans le groupe composé de copolymère d'éthylène vinylacétate et copolymère d'éthylène éthylacrylate.

4. Procédé de fabrication de papier stratifié contenant un agent actif selon l'une quelconque des revendications 1 à 3, dans lequel l'agent actif est un agent actif volatil.

5. Procédé de fabrication de papier stratifié contenant un agent actif selon la revendication 4, dans lequel l'agent actif volatil est au moins l'un des éléments choisis dans le groupe composé de parfums, agents anti-mauvaises odeurs, désodorants, agents fongicides, antiseptiques, germicides, inhibiteurs de corrosion, antimites, insecticides, répulsifs, rodenticides et antipourrissement.

Rodentiziden und Fäulnis verhindernden Mitteln, ist.

## Ansprüche

1. Verfahren zur Herstellung eines einen Wirkstoff enthaltenden Mehrschichtpapiers durch Imprägnieren des Papiers mit einem Wirkstoff, gekennzeichnet durch folgende Stufen :

   Auftragen eines flüssigen Wirkstoffes, der in einem Lösungsmittel gelöst werden kann, auf die Papierschicht eines Mehrschichtpapiers und Imprägnieren der Papierschicht mit diesem, wobei das Mehrschichtpapier aus der Papierschicht und einer thermoplastischen Polymerschicht, die mit dem Wirkstoff verträglich und auf eine Seite der Papierschicht laminiert ist, besteht, und

   Aufrollen oder Übereinanderlegen des imprägnierten Mehrschichtpapiers während einer vorbestimmten Zeit,

   wodurch bewirkt wird, daß zumindest ein Teil des flüssigen Wirkstoffes in die thermoplastische Polymerschicht übergeht und in sie eindringt.

2. Verfahren zur Herstellung eines einen Wirkstoff enthaltenden Mehrschichtpapiers nach Anspruch 1, in dem das thermoplastische Polymer, das mit einem Wirkstoff verträglich ist, ein Carboxylgruppen enthaltendes Ethylen-Copolymer oder ein Verbundmaterial aus einem Carboxylgruppen enthaltenden Ethylen-Copolymer und anderen Polymeren ist.

3. Verfahren zur Herstellung eines einen Wirkstoff enthaltenden Mehrschichtpapiers nach Anspruch 2, in dem das Carboxylgruppen enthaltende Ethylen-Copolymer mindestens ein Copolymer, ausgewählt unter Ethylen-Vinylacetat-Copolymer und Ethylen-Ethylacrylat-Copolymer, ist.

4. Verfahren zur Herstellung eines einen Wirkstoff enthaltenden Mehrschichtpapiers nach einem der Ansprüche 1 bis 3, in dem der Wirkstoff ein flüchtiger Wirkstoff ist.

5. Verfahren zur Herstellung eines einen Wirkstoff enthaltenden Mehrschichtpapiers nach Anspruch 4, in dem der flüchtige Wirkstoff mindestens ein Mittel, ausgewählt unter Parfums, geruchshemmenden Mitteln, Desodorantien, Pilzbefall verhütenden Mitteln, antiseptischen Mitteln, Desinfektionsmitteln, Rostinhibitoren, Mottenschutzmitteln, Insektiziden, Repellents,